# EUROPEAN PATENT SPECIFICATION

(11) **EP 0 650 713 B1**
(45) Date of publication and mention of the grant of the patent: **15.09.1999**
(21) Application number: 94308014.3
(22) Date of filing: 31.10.1994
(51) Int. Cl.: A61F 13/00, A61F 15/00

(54) **Wound packing and package therefore**
Wundverband und seine Verpackung
Pansement et son emballage

(30) Priority: 01.11.1993 US 144003; 06.09.1994 US 300745; 23.09.1994 US 310971
(43) Date of publication of application: 03.05.1995
(73) Proprietor: Paul Hartmann Aktiengesellschaft, 89522 Heidenheim (DE)
(72) Inventor: Cartmell, James V., Xenia, Ohio 45385 (US); Sturtevant, Wayne R., Centerville, Ohio 45458 (US); Wolf, Michael L., West Milton, Ohio 45383 (US)
(74) Representative: Dreiss, Fuhlendorf, Steimle & Becker, Patentanwälte

(56) References cited:
- FR-A- 2 552 670
- US-A- 2 004 614
- US-A- 3 152 694
- US-A- 4 281 650
- US-A- 4 296 179
- US-A- 4 842 597
- US-A- 5 059 424
- US-A- 5 154 706

## Description

The present invention relates to a sterile wound packing and a package therefore, and, more particularly to a wound packing in the form of a gauze or other similar absorbent material having a hydrogel material impregnated therein for absorbing wound exudate and to a package for dispensing the wound packing.

Secreting skin wounds, such as decubitus ulcers and open surgical wounds, have long presented a medical challenge in keeping such wounds sterile and relatively dry. Deep wounds provide an even greater challenge. The accumulation of wound exudate, such as blood, pustulation, and other wound fluids, in wound crevices, promotes growth of bacteria and other organisms which cause infection and delay the healing process. Such wound exudate may also cause maceration of tissue adjacent the wound and support infection thereof.

However, since it is often desirable to allow a wound to heal in a slightly "moist" or occlusive state, which is believed to accelerate healing, excess wound exudate must be removed. If excess wound exudate remains in or on a wound, a "blister" of exudate can form under the wound dressing which is not only unsightly, but also may cause the dressing to leak, thereby defeating the aim of sterility. However, existing methods of aspiration can lead to wound infection or can destroy sterility. Additionally, it is not desirable to remove all the exudate as that would result in a "dry" wound resulting in a slower healing process.

There is a substantial body of prior art relating to wound and/or surgical dressings or packings for treating skin lesions, such as decubitus ulcers and open wounds. In some instances, the wound dressing or packing may be designed to be only temporary, such as the use of gauze to absorb blood and/or other wound exudate. In others, the wound dressing is designed to be more permanent in nature, remaining in place for several hours or days during the healing process. In yet other instances, the wound dressing material is designed to be biodegradable and to break down over an extended period of time as a wound heals.

Aqueous moisture absorbing materials, such as a hydrogel material with a polyethylene glycol liquid curing agent, as disclosed in Spence, U.S. Patent No. 4,226,232, have been used as dressings on a wound site, but cannot be sterilized by irradiation due to the formation of free radicals within the aqueous material. Another aqueous absorbing material used to absorb wound exudate is a hydrophilic polymer as disclosed in Rawlings et al, U.S. Patent No. 4,657,006. Rawlings et al disclose a wound dressing which comprises a hydrophilic polymer having moisture and vapor permeability characteristics. However, a problem with the Rawlings et al wound dressing is that the wound exudate absorbed by the hydrophilic polymer hardens or solidifies the polymer, allowing pockets to develop between the polymer and the wound, thereby providing an excellent environment for bacteria proliferation.

In addition, wound dressings used in the past have not been conducive for healing extremely deep wounds and wounds having irregular shapes. To that end, wound dressings and surgical sponges formed from gauze and foam materials have been used for many years in surgical practice. These sponges and wound dressings have attempted to retain both the advantages of thin, soft and flexible single layer dressings and the absorptive cushioning and insulating properties of thicker pad-like structures. As a result, the sponges and wound dressings have traditionally been formed of multiple layers of thin, soft, low-count gauze material which are unified along fairly widely separated lines usually extending longitudinally or transversely.

Although such wound dressings and surgical sponges have been found useful in the past, none have provided the capability of absorbing large amounts of wound exudate without inhibiting the healing of the wound to which they are contacted. Most all of these dressings, including gauze and sponges, adhere to the wound upon removal, thereby damaging the wounds to which they are attached. This in turn prolongs the healing of such wounds. It would therefore be desirable to have a wound dressing or packing having a structure which is thin, flexible and soft yet absorbs wound exudate in the same manner as the more thick pad-like wound dressings.

Cartmell et al, U.S. Patent No. 5,115,801, disclose a thin-film burn dressing containing an aqueous hydrogel material partially impregnated in a foam or nonwoven material which contacts the wound directly, thus preventing damage to the new tissue when the dressing is removed. However, such an aqueous hydrogel wound dressing contains a high percentage of water, and thus is not as readily absorbent as other wound dressings. In addition, due to the large water content of the aqueous hydrogel material, the burn dressing requires additional backing and adhesive layers to properly support the hydrogel material which leads to increased production costs.

US-Patent 5,154,706 concerns a wound dressing for a deep wound comprising a hydrogel impregnated layer. This layer is stored in a box-like tray which is covered by a protective layer. The hydrogel impregnated layer is in the form of a rather thick sponge-like woundpacking material to be inserted in a deep wound.

US-Patent 4,296,179 relates to a package for sterilized products comprising sealed first and second sheets with the sterilized product therebetween. A comparable package for a compress which is impregnated with a medicament being liquid, paste or gel is disclosed in US-Patent 4,281,650.

US-Patent 2,004,614 discloses a box-like container for absorbent cotton in rope or strip form which may be withdrawn therefrom in small amounts as it is needed. The rope or strip beeing stored as a 3-dimensional coil-form.

Accordingly, there is a need in the art for a wound packing which is capable of absorbing large amounts of wound exudate without inhibiting the healing of the wound to which it is contacted and without the need for additional support layers. There is also a need for a wound dressing which has a pliable, soft structure so as to permit the wound dressing to be readily applied to wounds having irregular shapes and depths. Finally, there is a need for such a wound dressing which can be conveniently packaged and dispensed while maintaining its sterility.

The present invention meets those needs by providing a sterile wound packing material and package therefor. The wound packing is in the form of a gauze or similar absorbent material and may have a partially or substantially dehydrated hydrogel material impregnated therein for absorbing wound exudate. The wound packing is capable of readily absorbing large amounts of wound exudate without requiring additional supporting layers or structure. The wound packing does not adhere to the wound and is readily removed without inhibiting the healing of the wound to which it is contacted. In addition, the present wound packing is soft and pliable, which permits the wound packing to be readily applied to wounds having irregular shapes and depths. Further, in embodiments where the hydrogel still contains some amount of water, the wound dressing provides a cooling effect on the wound and surrounding areas of a patient's skin due to the evaporation of moisture from the hydrogel in use.

According to one aspect of the present invention, a sterile wound packing is provided and includes a sterile, wound packing and a package therefore. The wound packing includes an absorbent layer capable of absorbing wound exudate. In a preferred embodiment, the wound packing is in the form of a substantially flat, coiled, spirally-cut layer. The absorbent layer of the wound packing is preferably selected from the group consisting of woven gauze, fabric, nonwoven natural or synthetic fibers, polymeric sheets and films, and the like.

In certain embodiments of the invention where it is desired that the wound packing remain in place for an extended period, the absorbent layer is impregnated with a hydrogel, which may be a partially dehydrated hydrogel containing from about 5 to 55% by weight water, and more preferably, from about 40% to about 45% by weight water. By partially dehydrated, it is meant that at least a portion, but not all of the water has been removed from the hydrogel material. Preferably, the absorbent layer includes interstices, and the partially dehydrated hydrogel material is completely impregnated in the interstices such that the hydrogel material is substantially exposed at the outer surface of the absorbent layer.

In this way, the partially dehydrated hydrogel material is substantially in contact with the wound while contact of the absorbent layer thereto is minimized so as to preclude the absorbent layer from adhering to the wound. This is desirable since healing of the wound is inhibited when the absorbent layer sticks or otherwise adheres to the new cell tissue forming in the wound. This arrangement is also desirable in that the partially dehydrated hydrogel material provides a desirable cooling effect when the wound packing is applied to a patient's skin due to the evaporation of water from the hydrogel in use. It has also been found that the partially dehydrated hydrogel material adheres sufficiently to the absorbent layer and has sufficient structural integrity so as to eliminate the need for backing or adhesive layers to provide additional support for the hydrogel material.

In an alternative embodiment of the invention, the absorbent layer of the wound packing is impregnated with a substantially dehydrated hydrogel containing less than 5% by weight water. The substantially dehydrated hydrogel is preferably impregnated in the interstices of the absorbent layer as described above such that the hydrogel material is substantially exposed at the outer surface of the absorbent layer. Because the wound packing contains little or no water, it can also be used without the use of any additional support layers.

The package for the wound packing includes sealed first and second sheets with the flexible wound packing therebetween. The first and second sheets are made of any suitable material capable of maintaining the sterility of the contents of the package such as metal foil, metal-coated polymer, polymer, paper, or coated paper.
The package is preferably opened by peeling apart the first and second sealed sheets. Preferably, a portion of the package is sealed such that a free-lifting edge or corner is provided to facilitate peeling apart the sheets.

In embodiments where the wound packing is in the form of a substantially flat, spirally-cut layer, the package may be designed to be opened by puncturing one of the sheets to form an opening in the package. The spirally-cut packing may then be removed by pulling on one end thereof out through the opening. The wound packing may be cut to length as needed or may be designed to be packaged in different widths and lengths.

To aid in opening the package, the package may include means for stiffening the package to promote easy puncture. The stiffening means comprises card stock or a stiff polymeric sheet disposed in the package. Alternatively, the stiffening means comprise a release liner on the flexible material. In another embodiment, one or both of the sheets forming the package may include a weakened area, again to aid in opening the packing. The weakened area may comprise, for example, a pattern of score lines or perforations which can be readily broken to facilitate access to an end of the spirally-cut wound packing in the package. However, in order to maintain sterility of the package, the score lines or perforations should not penetrate the package.

In another embodiment, the package includes an opening in one of the first and second sheets located adjacent one end of the packing material for providing access to the packing material, and removable means for sealing the opening. Again, to aid in the opening of the package, there may be means for stiffening the area around the opening such as card stock laminated to an inner surface of the package around the opening. The opening may be located substantially at the center of the package adjacent one end of the spirally-cut wound packing or, alternatively, near an edge of the package adjacent the opposite end of the wound packing.

In embodiments where the wound packing is spirally cut, the package also preferably includes a backing layer adhered to a portion of the inner surface of one of the first and second sheets, with the wound packing positioned on the backing layer. Preferably, the backing layer comprises a polymer such as polyester which includes a release coating on its surface. The wound packing is positioned on the backing layer such that it contacts the release coating. The backing layer prevents the wound packing from shifting inside the unopened package, and, once the package has been opened, supports the packing while it is being dispensed.

The package also preferably includes means for protecting the wound packing from contamination prior to dispensing it from the opened package comprising a release liner or card stock which overlies the wound packing inside the package.

Accordingly, it is a feature of the present invention to provide a wound packing which is capable of readily absorbing large amounts of wound exudate without inhibiting the healing of the wound to which it is contacted and without the need for additional support layers. It is also a feature of the invention to provide a wound packing which possesses a pliable, soft structure for application to wounds having irregular shapes and depths. Finally, it is a feature of the present invention to provide a package which allows the wound packing to be easily dispensed and stored indefinitely in a compact, sterile condition. These, and other features and advantages of the present invention will become apparent from the following detailed description, the accompanying drawings, and the appended claims.

In order that the invention may be more readily understood, reference will now be made by example to the accompanying drawings, in which:
Fig. 1 is a top view of a wound packing in accordance with the present invention;
Fig. 1A is a top view of a wound packing illustrating the spiral-cut configuration;
Fig. 2 is a perspective view of a sealed package containing the wound packing with a free-lifting edge for peeling;
Fig. 2A is an alternative embodiment of the package shown in Fig. 2;
Fig. 3 is an exploded perspective view of the wound packing and package of Fig. 2A;
Fig. 3A is a variation of the embodiment shown in Fig. 3;
Fig. 3B is an exploded perspective view of a wound package including a backing layer and a release layer;
Fig. 4 is a perspective view of a package containing a spiral cut wound packing being removed thruugh a centrally located access hole after removal of the sealing tab;
Fig. 4A is a perspective view of a package containing a spiral cut wound packing being peeled away from a backing layer of the packaging;
Figs. 5 and 5A are enlarged, partially cut away perspectives of various applications of the wound packing of the present invention;
Figs. 6 and 6A are schematic views illustrating a process by which the wound packing and package can be made;
Fig. 7 illustrates one embodiment of the wound packing after it has been applied to a draining wound; and
Fig. 8 is a cross-sectional view of the wound packing of Fig. 7.

The present invention provides a wound packing in the form of an absorbent material which may have a partially or substantially dehydrated hydrogel material impregnated therein for absorbing wound exudate. The use of a partially or substantially dehydrated hydrogel material provides several advantages over previously used wound dressings. Because at least a portion of the water has been removed from the hydrogel material, the wound packing more readily absorbs fluids from the wound than aqueous hydrogel wound dressings. For the embodiment using a partially dehydrated hydrogel material, the hydrogel still contains enough water such that a desired cooling effect results when the wound dressing contacts a patient's skin. The use of a partially or substantially dehydrated hydrogel also provides the advantage that the hydrogel is sufficiently supported by the gauze or other absorbent material without the need for additional support layers, structure, or packing materials.

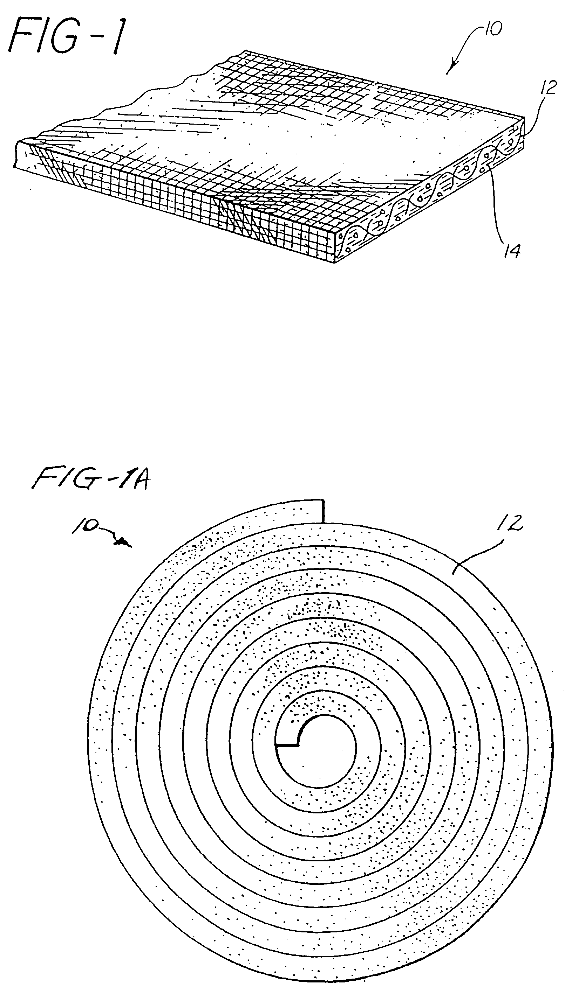

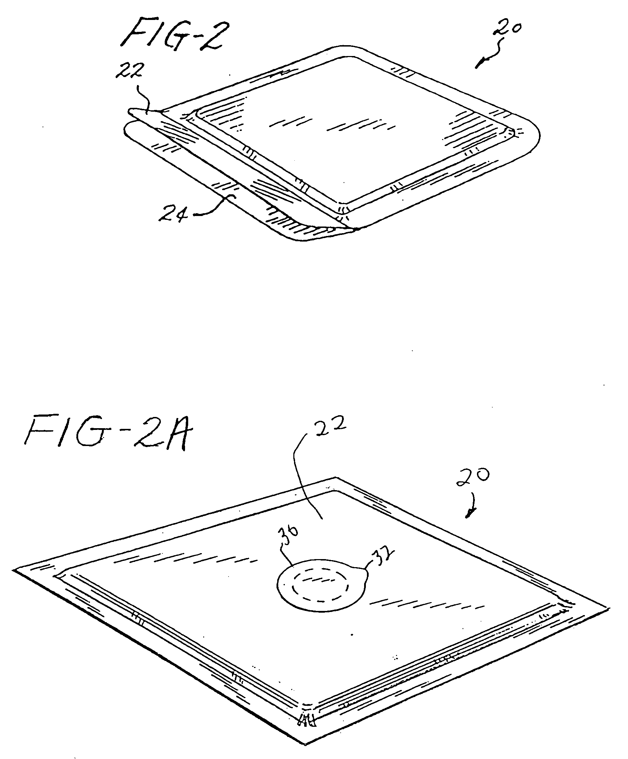

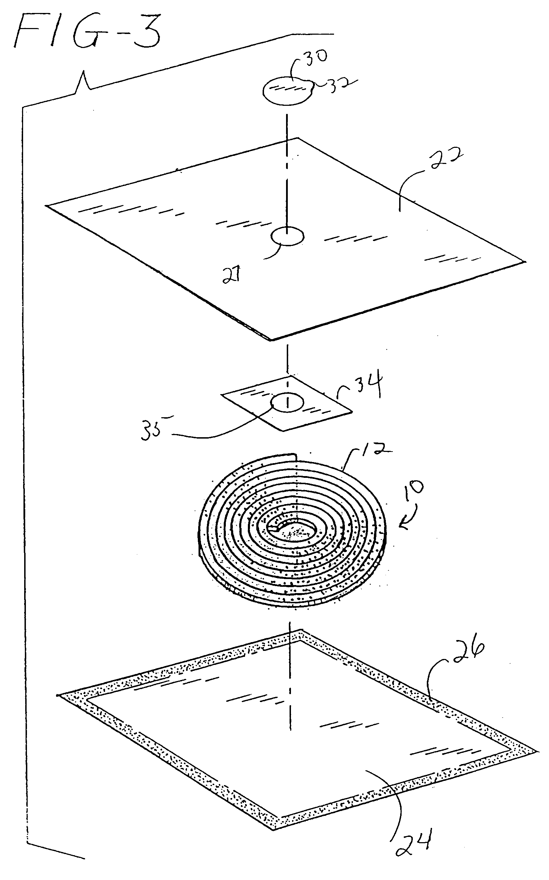

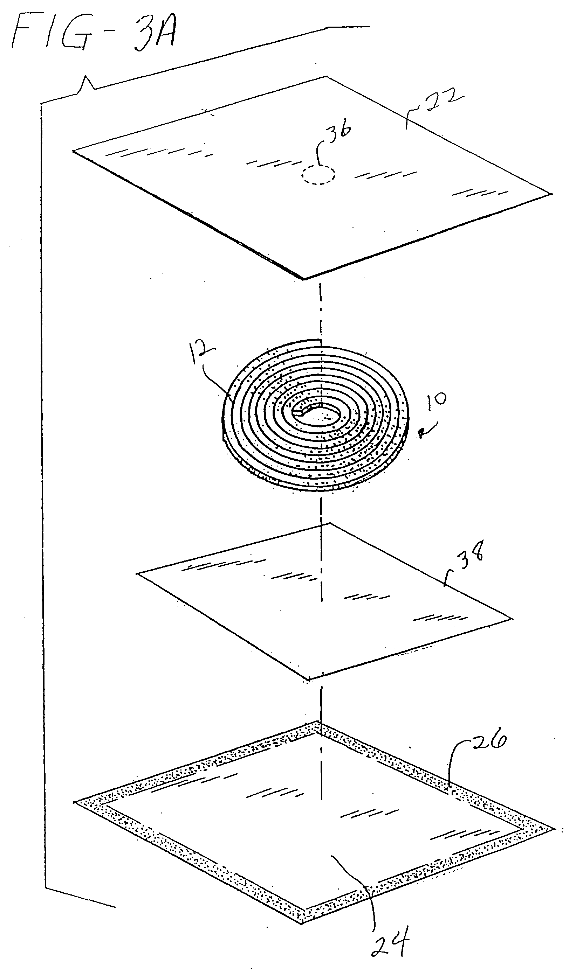

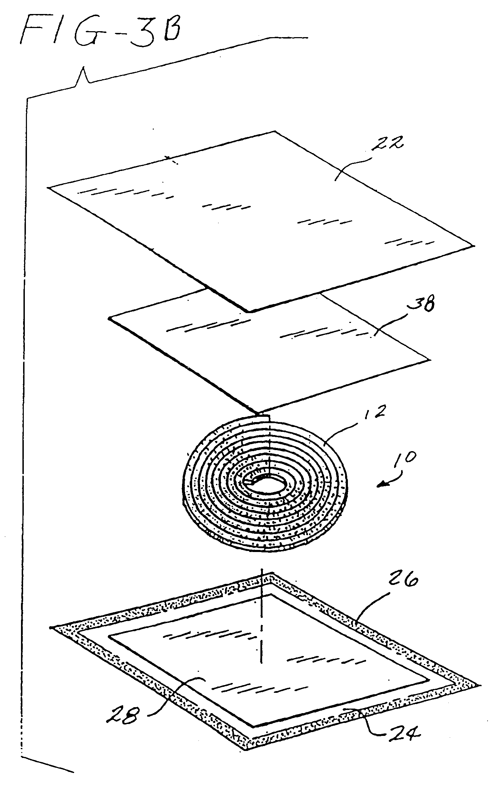

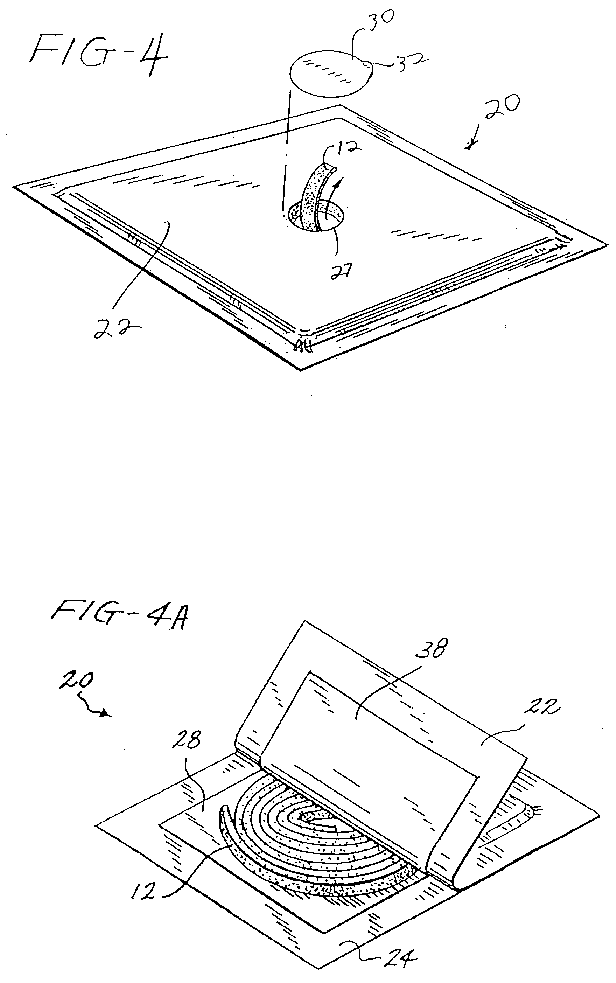

As shown in Fig. 1, the present invention provides a wound packing 10 in the form of a thin, pliable, absorbent structure suitable for use in the treatment of wounds on a patient. The wound packing 10 comprises a flexible absorbent layer 12 which may optionally have a partially or substantially dehydrated hydrogel material 14 impregnated therein for absorbing wound exudate. While those skilled in the art will appreciate the difficulty in illustrating the presence of a hydrogel material 14 in absorbent layer 12, it should be understood that the partially or substantially dehydrated hydrogel material when used is preferably completely impregnated in the interstices between fibers of the absorbent layer 12.

It is preferred that the absorbent layer 12 be formed of material which is capable of absorbing wound exudate and or blood cells, as well as being capable of supporting the optional hydrogel material 14. Those skilled in the art will appreciate that materials having interstices within which materials may be absorbed or impregnated are particularly suitable for such purposes.

The partially or substantially dehydrated hydrogel material 14 must be able to adhere to the absorbent layer 12 so as to form a pliable, thin structure which, when contacted with a draining wound on a patient, absorbs large amounts of wound exudate without inhibiting the healing of such wound.

Examples of suitable materials for absorbent layer 12 include woven gauze, natural or synthetic fabrics, nonwoven natural or synthetic fibers (e.g., felts), or polymeric sheets and films to the extent that they can be fabricated to include interstices and be absorbent. Absorbent layer 12 can comprise a single fibrous layer, a plurality of layers stacked one atop the other, or a felt-like nonwoven material. Typically, the material of absorbent layer 12 will have a thickness of from several mils to a few hundred mils (about .076 mm to 7.6 mm).

As shown in Fig. 1A, wound packing 10 may also be in the form of a substantially flat, coiled spiral-cut layer of material. The material may be manufactured in the form of a continuous web which can then optionally be impregnated with a hydrogel and cut into desired sizes. For example, an approximately 13 cm diameter disk of absorbent material may be spiral cut into a continuous 0.6 cm width strip to provide about 2 meters of wound packing. Similarly, an approximately 20 cm diameter disk of absorbent material may be spiral cut into a continuous 2.5 cm width strip to provide about 1 meter of wound packing. Those skilled in this art will recognize that there are many combinations of widths and lengths of the wound packing material which can be manufactured. Because of the spiral cut design, the wound packing may be easily packaged compactly and in a variety of widths and lengths.

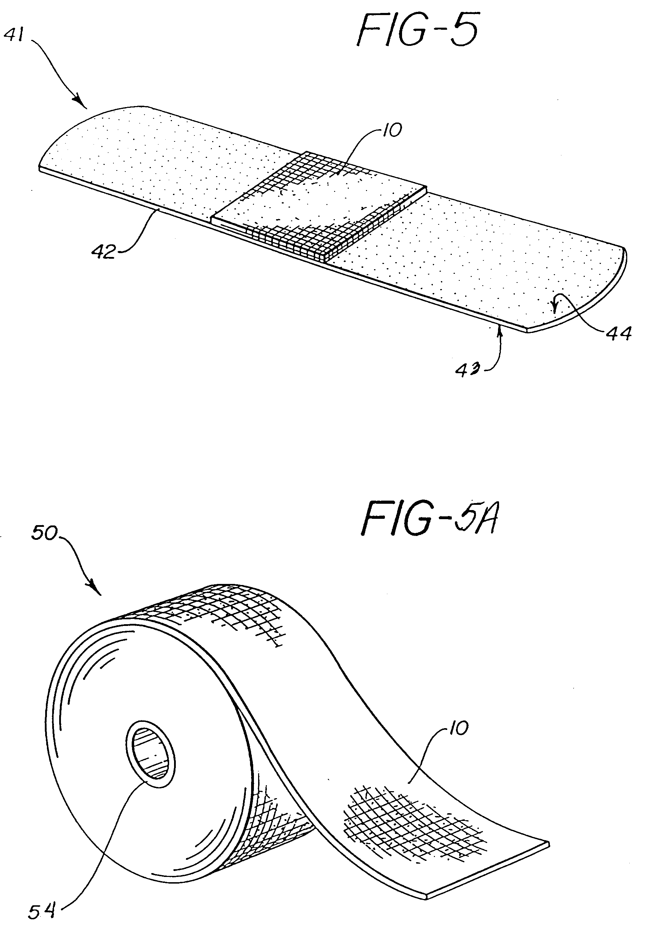

Referring now to Fig. 2, a package 20 is provided for the wound packing. Package 20 includes peripherally sealed first and second sheets 22, 24 which contain wound packing 10 (not shown) therebetween. First and second sheets 22, 24 may be made from any suitable material capable of maintaining the sterility of the wound packing 10 within the package. Examples of suitable sheet material include metal foils, metal coated polymer sheets, polymers (including but not limited to those which provide moisture and/or gas barrier properties), paper, or coated paper. Where an optional partially dehydrated hydrogel is impregnated into the wound packing, it is preferred that packaging with moisture barrier properties be used to insure a long shelf life for the package.

As shown in Fig. 2, one side portion of the perimeter of the package is sealed so that the edges of the two sheets are free-lifting and may be peeled apart. It will be appreciated that other variations of this embodiment may also be employed. For example, the package can be configured so that the sheets include a free-lifting corner section of the package. If desired, a tab may be included between the two sheets along an edge or in a corner to facilitate grasping and peeling apart of the sheets. The package may also include a cut or notch along an edge which will facilitate tearing for opening of the package.

The sheets may be heat sealed together or adhesively sealed, such as by a bead of peripheral adhesive 26 (shown in Fig. 3) on one or both sheets. Alternatively, any sealing process which results in an air-tight seal to maintain the sterility of the wound packing within the package is suitable.

In an alternative embodiment shown in Figs. 2A and 4, package 20 may be designed to be opened by puncturing one or both of sheets 22, 24 to form an opening through which one end of absorbent layer 12 may be pulled.

In the embodiment shown in Figs 2A and 3, package 20 includes a precut opening 27 in sheet 22 which is located adjacent one end of the spirally-cut wound packing. In Figs. 2A and 3, this opening is shown as being centrally located on the package over the end of the wound packing nearest the center of absorbent layer 12. Alternatively, the opening may be located over the opposite end of the wound packing. While being shown for illustration purposes as being circular, the size and shape of the opening is not critical so long as ready access to one end of the wound packing 10 is provided.

Package 20 includes removable means for sealing the opening such as a tab 30 which may be adhered or otherwise sealed over opening 27. If a pressure sensitive adhesive is used to seal the tab over opening 27, then the tab may also be used to reseal the opening if desired after a desired length of wound packing 10 has been removed and cut. Tab 30 may include a free or clean-lifting edge 32 which may be grasped to facilitate its removal. Once tab 30 has been removed, an end of absorbent material 12 may be pulled from the opening 27 as shown in Fig. 4 to a desired length or amount. The flexible material may then be cut. Alternatively, the entire contents of package 20 may be removed and used at the same time.

As shown in Fig. 3, package 20 may also include a stiffening means therein to promote easy puncture or opening of the package. Such stiffening means may comprise card stock 34 or a stiff polymeric sheet disposed in the package. Card stock 34 may itself include a corresponding opening 35 and be positioned or secured to the underside of sheet 22 beneath opening 28.

In an alternative embodiment of the invention shown in Fig. 3A, where like reference numerals represent like elements, sheet 22 includes a weakened area 36 to aid in the opening of package 20. Weakened area 36, as shown, comprises a series of score lines or perforations which can be readily broken to facilitate to access to an end of absorbent layer 12. Again, the pattern and size of the weakened area is not critical as long as ready access to an end of wound packing 10 is provided. However, care should be taken so that the score lines or perforations do not penetrate completely into the package and cause the packing to lose its sterility. In this embodiment shown in Fig. 3A, a release liner 38 underlies flexible absorbent layer 12. Release liner 38 may also act as the stiffening means to promote easy opening of package 20.

In yet another alternative embodiment shown in Fig. 3B, the package includes a backing layer 28 which is adhered to the inner surface of sheet 24. The backing layer preferably comprises a polymer such as polyester or polyethylene although other suitable materials such as paper may be used. Polyester is the preferred backing layer for the present invention as it is a strong film which is easy to kiss-cut for manufacturing purposes as will be explained in greater detail below. The backing layer is coated on its surface with a release coating such as silicone, and is coated on its opposite surface with a pressure sensitive adhesive which adheres to the inner surface of sheet 24. The wound packing 10 is positioned on the backing layer 28 such that it contacts the release coating. Once adhered to the inside of the package, the backing layer supports the wound packing in position until it is dispensed.

The package also preferably includes means for protecting the wound packing from contamination prior to dispensing which comprises a release liner 38. The release liner preferably comprises polyethylene and may include a release coating on the side of the liner which contacts wound packing 10. Card stock, preferably coated, may also be used as an alternative means of protection.

Referring now to Fig. 4A, once the first and second sheets 22 and 24 have been peeled apart, and release liner 38 has been peeled away from the wound packing, an end of absorbent material 12 may be peeled away from the backing layer 28 to a desired length or amount. The absorbent material 12 may then be cut. Alternatively, the entire contents of package 20 may be removed and used at the same time.

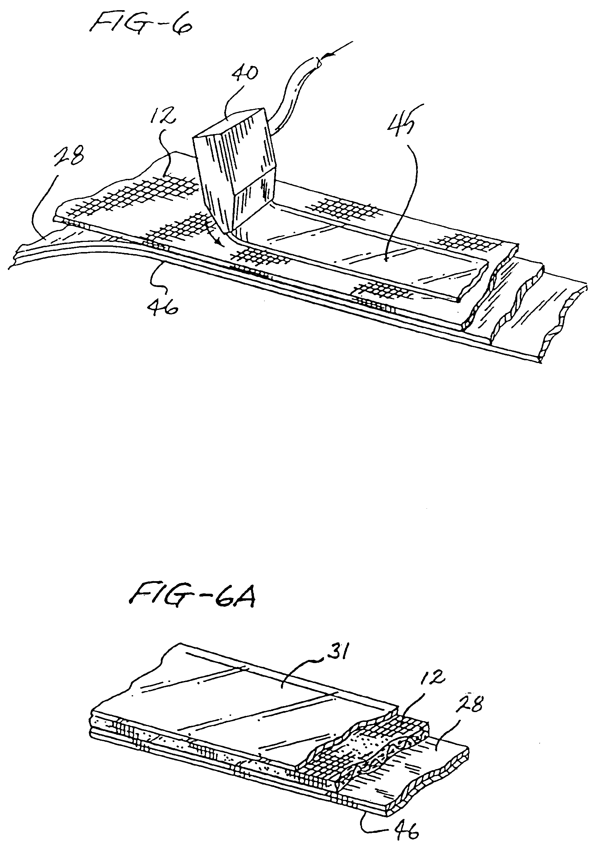

For purposes of providing a more intuitive understanding of the wound packing 10 and package 20, one process by which the wound packing 10 and package can be made is schematically illustrated in Figs. 6 and 6A. As seen in Fig. 6, a backing sheet 28, preferably with a release coating on its surface, is advanced on a feed line. The sheet 28 has a pressure sensitive adhesive on its opposite surface which is covered with a release liner 46.

An absorbent layer 12 is also fed in continuous web form onto the feed line so that the web is in contact with the release coated surface of the backing sheet 28, such as, for example, a silicone coated polyester. The absorbent layer 12 is fed under an applicator 40 capable of receiving and applying a liquid or uncured hydrogel material 45 from a source (not shown) without permitting it to cure within its components. Applicator 40 applies uncured hydrogel material 45 onto absorbent layer 12 in an amount sufficient to impregnate the interstices therein and provide exposure of the hydrogel at the outer surfaces of layer 12. As those skilled in the art will appreciate, the amount of uncured hydrogel material 45 applied will vary with the particular material used as absorbent layer 12 and the width and thickness of the web to be coated.

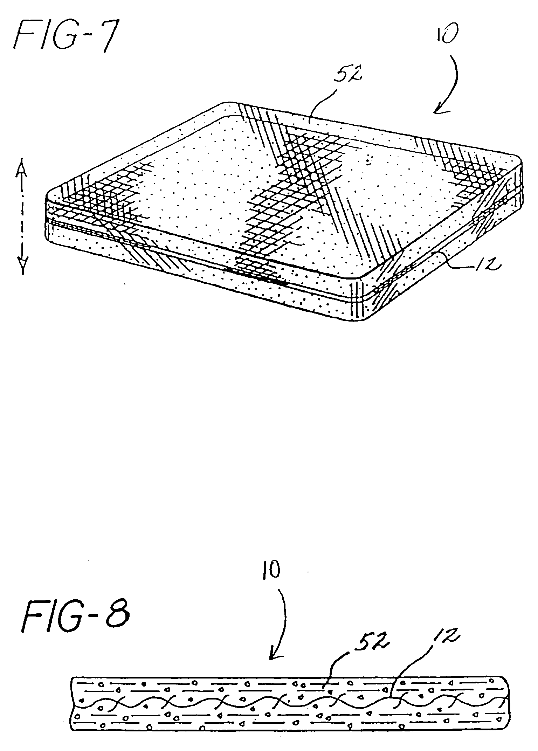

Uncured hydrogel material 45 is then allowed to cure downstream on the feed line to form a hydrated hydrogel material 52 surrounding and impregnating absorbent layer 12 (Fig. 8). Thereafter, the wound packing 10 is partially dried, oven-baked or otherwise partially dehydrated so as to evaporate a portion of the water contained in the hydrogel material 52. Typically, 50% by weight or more of the water initially in the hydrogel will be removed.

However, it should also be appreciated that the wound packing may be dried or oven-baked so that all of almost all of the water contained in the hydrogel material 52 is evaporated. The resulting substantially dehydrated hydrogel material should contain less than 5% by weight water. While such a hydrogel material is capable of absorbing large amounts of wound exudate, the resulting wound packing becomes somewhat stiffer due to the dryness of the hydrogel. For this reason, it is preferred that the hydrogel material is only partially dehydrated as described above so that it contains from about 5% to about 55% by weight water, and more preferably, from about 40% to about 45% by weight water.

Once the absorbent layer has been impregnated with hydrogel, a release coated polymer film 31 is laminated to the top of the hydrogel-impregnated absorbent layer 12 such that the absorbent layer is sandwiched between sheets 31 and 28 as shown in Fig. 6A.

Thereafter, if desired, the continuous web of wound packing 10 may be cut into individual sheets. According to the present invention, the wound packing is then be spiral cut to the form shown in Fig. 1. Spiral cutting is accomplished using a kiss-cut die, where the die cuts through the release-coated film 31 and the hydrogel impregnated absorbent layer 12, but not the backing sheet 28. After cutting, film 31 is then removed, and a fresh sheet of a release-coated material such as silicone coated polyethylene is laminated to the spiral-cut wound packing. This sheet of polyethylene remains on the wound packing as release liner 38.

The polyethylene and polyester sheets are then die cut so that both sheets extend beyond the edges of the wound packing. The release liner 46 is then removed from the adhesive coated surface of backing layer 28. The backing layer 28 may then be adhered to the inner surface of one of the sheets of the package, with the spiral cut wound packing sandwiched between the backing layer 28 and the release liner 38. The first and second sheets of the package are then adhered together to form a sealed package 20 as shown in Fig. 2.

Referring now to Fig. 7, which has been greatly enlarged for purposes of illustration, a small piece of wound packing 10, after having been contacted with a draining wound, is illustrated. Where a partially or substantially dehydrated hydrogel has been used, Fig. 7 illustrates the expansion or swelling (as depicted by the arrows in dotted lines) of the hydrogel material upon acquisition of bodily fluids, such as wound exudate, from the wound to which the wound packing 10 is applied. The expanded or hydrated hydrogel material is referred to herein by reference numeral 52. Wound packing 10, when it contains optional hydrogel, is therefore analogous to a sponge in that its initial partially or substantially dehydrated state expands as fluids are absorbed.

Fig. 8 may also be considered to illustrate a cross-sectional view of a small piece of wound packing 10 depicted in Fig. 7 and shows hydrogel material 52 swelled in and around absorbent layer 12. As those skilled in the art will appreciate, hydrogel material 52 depicted in Fig. 7 is the same as the cured hydrogel material 52 discussed with respect to the process by which wound packing 10 is made and illustrated in Fig. 6. In essence, cured and hydrated hydrogel material 52 may be initially partially or substantially dehydrated for packaging and then returned to its original hydrated state upon wound exudate absorption.

The preferred hydrogel material for use in the present invention is formed from an aqueous mixture of polyhydric alcohol, an aliphatic diisocyanate terminated prepolymer, polyethylene oxide based diamine and sodium chloride. Such an aqueous hydrogel material is taught in Cartmell et al, U.S. Patent No. 5,115,801, and contains approximately 61% water by weight when fully hydrated. Preferably, the polyhydric alcohol is selected from the group consisting of polypropylene glycol, polyethylene glycol and glycerine. After curing the mixture, at least a portion of the water is removed from the resulting hydrogel as described above to form the partially or substantially dehydrated hydrogel material.

The hydrogel material 52 in its partially or substantially dehydrated state, which is referred to herein as hydrogel material 14, provides a highly absorbent material capable of retaining large amounts of wound exudate, thereby rendering it very suitable for use in wound dressings. By forming the hydrogel material 14 from the aforementioned aqueous mixture and then removing water, the wound dressing 10 remains intact as it absorbs wound exudate from the wound.

Moreover, the preferred hydrogel material does not adhere or stick to the wound thereby allowing for easy removal of wound packing 10 substantially as a single piece. Additionally, the biocompatibility of the hydrogel material within the wound is extremely favorable. Thus, the resulting hydrogel material 52, and therefore the dehydrated hydrogel material 14, provides a bio-compatible, non-irritating, fluid absorbing, bacterial protective, cushioning, skin-like media over the wound site. An additional advantage of the hydrogel material 52 is that it may be transparent, rendering it possible to inspect the wound site through absorbent layer 12 without removing wound packing 10.

The preferred aliphatic diisocyanate terminated prepolymer is an isophorone diisocyanate terminated prepolymer based on polyols containing more than about 40% polyethylene oxide and having an isocyanate content of about 3% by weight. The molecular weight of the isophorone diisocyanate terminated prepolymer is preferably in a range from about 1500 to about 8000 and most preferably, from about 4000 to about 5000. The polyethylene oxide based polyamine is preferably a polyethylene oxide based diamine having a molecular weight in a range from about 200 to about 6000 and most preferably, about 2000. It is also preferable that the aliphatic diisocyanate terminated prepolymer and the polyethylene oxide based polyamine have a stoichiometric ratio of about 1:1. Those skilled in the art will appreciate that all of the constituents with the preferred hydrogel material may be readily synthesized or purchased commercially neither of which is more preferred.

It has been found that a more preferred hydrogel material 52, and therefore the dehydrated hydrogel material 14, is formed from an aqueous mixture including from about 0% to about 90% by weight polyhydric alcohol; from about 6% to about 60% by weight aliphatic diisocyanate terminated prepolymer; from about 4% to about 40% by weight polyethylene oxide based polyamine; up to about 2% by weight sodium chloride; and the balance water. A more preferred hydrogel composition for forming the hydrogel material 52 is formed from a mixture comprising from about 15% to about 30% by weight polypropylene glycol; from about 8% to about 14% by weight isophorone diisocyanate terminated prepolymer; from about 5% to about 10% by weight polyethylene oxide based diamine; and up to about 1% by weight sodium chloride; and the balance water. Most preferably, the hydrogel material 52 is formed from a mixture comprising: (a) from about 16% to 17% by weight polypropylene glycol; (b) from about 10% to 12% by weight isophorone diisocyanate terminated prepolymer; (c) from about 7% to 9% by weight polyethylene oxide based diamine; (d) about 0.5% to 1% by weight sodium chloride; and (e) the balance water.

The aforementioned preferred hydrogel compositions provide a wound packing 10 having the desired properties of excellent biocompatibility and absorption of exudate properties without adhering to the wound. However, other materials having such characteristics, including but not limited to the aforementioned hydrogel compositions, may be used to form the hydrogel material 52 in accordance with the present invention.

While the wound packing 10 of the present invention may serve as an extremely viable wound packing by itself, the wound packing 10 may also be incorporated into other wound packings in order to improve their performance. By way of example only, a few of such wound packing embodiments are illustrated in Figs 5 and 5a. Fig. 5 illustrates the use of the wound packing in a bandage 41 of the self-adhesive type. The self-adhesive bandage 41 comprises a substrate 42 having first and second sides 44 and 43, respectively, wherein the side 44 contacts a patient and includes a pressure sensitive adhesive coated onto at least one portion of side 44.

As seen in Fig. 5, the bandage 41 has pressure sensitive adhesive coated over all portions which ultimately contact the patient. The bandage 41 also includes the wound packing 10 secured to the side 44 for contacting a wound on the patient. Preferably, the wound packing 10 performs and is formed as described above. In this way, the wound dressing 10 in the bandage 41 swells as it absorbs wound exudate from the wound to which it is attached.

Turning now to Fig. 5a, the wound packing 10 is illustrate din the form of a roll dispenser 50. The wound packing 10 is formed as described above and then wrapped around a spool 54 or similar device such that the wound packing 10 can be easily accessed. As a result, the roll dispenser 50 facilitates quick and easy access to the wound packing 10 in that a user can cut a sufficient amount of the wound packing 10 to treat a wound on a patient. It should now be apparent to those skilled in the art that the wound packing 10 can be used in a wide variety of existing wound dressings as well as on its own.

## Claims

1. A wound packing comprising:
a sterile wound packing material (10) including a flexible, substantially flat absorbent layer (12) capable of absorbing wound exudate; and
a package (20) for said wound packing material (10), said package (20) comprising sealed first and second sheets (22, 24) with said wound packing material (10) therebetween, **characterized in** that said absorbent layer (12) is in the form of a coiled spirally-cut layer.

2. A sterile wound packing as recited in claim 1 wherein said absorbent layer (12) is impregnated with a partially dehydrated hydrogel (14) containing from about 5 to about 55 % by weight water.

3. A sterile wound packing as recited in claim 1 wherein said absorbent layer (12) is impregnated with a substantially dehydrated hydrogel (14) containing less than 5 % by weight water.

4. A sterile wound packing as recited in claims 1 or 2 wherein said absorbent layer (12) includes interstices, and said partially dehydrated hydrogel material (14) is completely impregnated in said interstices such that said hydrogel material (14) is substantially exposed at the outer surface of said absorbent layer (12).

5. A sterile wound packing as recited in claims 1 or 2 wherein said partially dehydrated hydrogel material (14) contains from about 40 % to about 45 % by weight water.

6. A sterile wound packing as recited in claim 1 in which said absorbent layer (12) is selected from the group consisting of woven gauze, fabric, nonwoven natural or synthetic fibers, polymeric sheets and films.

7. A sterile wound packing as recited in claim 1 wherein said package (20) includes a backing layer (28) having a release coating on its surface which is adhered to a portion of the inner surface of one of said first and second sheets, and wherein said wound packing material (10) is positioned on said backing layer (28) such that it contacts said release coating.

8. A sterile wound packing as recited in claim 1 in which said package (20) includes an opening (27) in one of said first and second sheets (22, 24) located adjacent one end of said wound packing material (10) for providing access to said wound packing material (10), and removable means (30) for sealing said opening.

9. A sterile wound packing as recited in claim 1 in which said first and second sheets (22, 24) of said package (20) are made of metal foil, metal-coated polymer, polymer, paper, or coated paper.

## Patentansprüche

1. Wundpackung mit:
einem sterilen Wundpackungsstoff (10) mit einer flexiblen, weitgehend flachen absorbierenden Lage (12), die zum Absorbieren von Wundexsudat in der Lage ist; und
einer Verpackung (20) für den Wundpackungsstoff (10), wobei die Verpackung (20) dichte erste und zweite Lagen (22, 24) mit dem Wundpackungsstoff (10) dazwischen besitzt, dadurch gekennzeichnet, daß die absorbierende Lage (12) in der Form einer zusammengerollten spiralförmig geschnittenen Lage vorliegt.

2. Sterile Wundpackung nach Anspruch 1, wobei die absorbierende Lage (12) mit einem teilweise dehydrierten Hydrogel (14) imprägniert ist, welches von etwa 5 bis etwa 55 Gewichtsprozent Wasser enthält.

3. Sterile Wundpackung nach Anspruch 1, wobei die absorbierende Lage (12) mit einem weitgehend dehydrierten Hydrogel (14) imprägniert ist, welches weniger als 5 Gewichtsprozent Wasser enthält.

4. Sterile Wundpackung nach Anspruch 1 oder 2, wobei die absorbierende Lage (12) Zwischenräume aufweist und der teilweise dehydrierte Hydrogelstoff (14) vollständig in den Zwischenräumen imprägniert ist derart, daß der Hydrogelstoff (14) weitgehend an der Außenfläche der absorbierenden Lage (12) angeordnet ist.

5. Sterile Wundpackung nach Anspruch 1 oder 2, wobei der teilweise dehydrierte Hydrogelstoff (14) von etwa 40 Gewichtsprozent bis etwa 45 Gewichtsprozent Wasser enthält.

6. Sterile Wundpackung nach Anspruch 1, bei der die absorbierende Lage (12) ausgewählt ist aus der Gruppe bestehend aus gewebter Gaze, Gewebe, nichtgewebten natürlichen oder synthetischen Fasern, polymeren Lagen und Filmen.

7. Sterile Wundpackung nach Anspruch 1, wobei die Verpackung (20) eine Trägerlage (28) aufweist mit einer Ablösebeschichtung auf ihrer Oberfläche, die an einem Bereich der Innenfläche von einer der ersten und zweiten Lagen haftet und wobei der Wundpackungsstoff (10) an der Trägerlage (28) angeordnet ist derart, daß er die Ablösebeschichtung berührt.

8. Sterile Wundpackung nach Anspruch 1, wobei die Verpackung (20) eine Öffnung (27) in einer der ersten und zweiten Lagen (22, 24) benachbart zu einem Ende des Wundpackungsstoffes aufweist zur Bildung eines Zugangs zu dem Wundpackungsstoff (10) und eine abnehmbare Einrichtung (30) zum Abdichten der Öffnung.

9. Sterile Wundpackung nach Anspruch 1, wobei die ersten und zweiten Lagen (22, 24) der Verpackung (20) aus einer Metallfolie, einem metallbeschichteten Polymer, einem Polymer, Papier oder beschichtetem Papier gefertigt sind.

## Revendications

1. Pansement comprenant :
- un matériau (10) de pansement stérile incluant une couche (12) absorbante, flexible et sensiblement plate, apte à absorber de l'exsudat de plaie ; et
- un emballage (20) pour ledit matériau (10) de pansement, ledit emballage (20) comprenant des première et seconde feuilles scellées (22, 24) avec ledit matériau (10) entre les deux,
caractérisé en ce que ladite couche (12) absorbante est sous la forme d'une couche enroulée et découpée en forme de spirale.

2. Pansement stérile selon la revendication 1, caractérisé en ce que ladite couche absorbante (12) est imprégnée d'un hydrogel (14) partiellement déshydraté contenant entre entiron 5 et environ 55% en poids d'eau.

3. Pansement stérile selon la revendication 1, caractérisé en ce que ladite couche absorbante (12) est imprégnée d'un hydrogel (14) sensiblement déshydraté contenant moins de 5% d'eau en poids.

4. Pansement stérile selon l'une des revendications 1 ou 2, caractérisé en ce que ladite couche absorbante inclut des interstices, et ledit matériau (14) d'hydrogel partiellement déshydraté est complètement imprégné dans lesdits interstices de façon que ledit matériau d'hydrogel (14) soit sensiblement exposé à la surface extérieure de ladite couche absorbante (12).

5. Pansement stérile selon l'une des revendications 1 ou 2, dans lequel ledit matériau d'hydrogel (14) sensiblement déshydraté contient entre environ 40% et environ 45% en poids d'eau.

6. Pansement stérile selon la revendication 1, caractérisé en ce que la couche absorbante (12) est choisie parmi le groupe consistant en gaze tissée, tissu, fibres synthétiques ou naturelles non tissées, feuilles et films polymères.

7. Pansement stérile selon la revendication 1, caractérisé en ce que ledit emballage (20) inclut une couche de support (28) ayant un revêtement d'enlèvement sur sa surface et qui adhère à une partie de la surface intérieure de l'une desdites première ou seconde feuilles, et en ce que le matériau de pansement (10) est disposé sur ladite couche de support (28) de manière qu'elle soit en contact avec ledit revêtement de relâchement.

8. Pansement stérile selon la revendication 1, caractérisé en ce que ledit emballage (20) inclut une ouverture (27), dans l'une desdites première ou seconde feuilles (22, 24), disposée de manière adjacente à une extrémité dudit matériau (10) de pansement pour permettre l'accès audit matériau (10) de pansement, et des moyens détachables (30) pour sceller ladite ouverture.

9. Pansement stérile selon la revendication 1, caractérisé en ce que lesdites première et seconde feuilles (22, 24) dudit emballage (20) sont réalisées sous forme de feuilles métalliques, de polymère à revêtement métallique, de polymère, de papier ou de papier couché.
